# EUROPÄISCHE PATENTSCHRIFT

(11) **EP 0 602 369 B1**
(45) Veröffentlichungstag und Bekanntmachung des Hinweises auf die Patenterteilung: **05.02.1997**
(21) Anmeldenummer: 93117855.2
(22) Anmeldetag: 04.11.1993
(51) Int. Cl.: B01J 10/00, B01J 19/24

(54) **Reaktorkonstruktionen für die Methylformiatsynthese**
Reactor constructions for methylformiate synthesis
Constructions de réacteur de synthèse de méthyle formiate

(30) Priorität: 05.11.1992 DE 4237369
(43) Veröffentlichungstag der Anmeldung: 22.06.1994
(73) Patentinhaber: Salzgitter Anlagenbau GmbH, 38239 Salzgitter (DE); BORISLAWER WISSENSCHAFTS- UND FORSCHUNGSINSTITUT BNII " SYNTHEZ", Borislaw 293 760 (UA)
(72) Erfinder: Sobotta, Georg Dr.-Ing., D-38239 Salzgitter (DE); Pazderski, Juri Antonowitsch Prof. Dr., UA-293760 Borislaw, Gebiet Lwow (UA); Skatschko, Wladimir Petrowitsch Dr., UA-293760 Borislaw, Gebiet Lwow (UA); Karajew, Rinald Anatoljewitsch Dr., AZ-370143 Baku (AZ); Tagajew, Oleg Alexejewitsch Dr., UA-293760 Borislaw, Gebiet Lwow (UA)
(74) Vertreter: Lüdtke, Frank

(56) Entgegenhaltungen:
- DE-A- 2 710 726
- DE-B- 1 009 749
- DE-B- 1 253 679
- SU-A- 816 525

## Beschreibung

Die Erfindung gehört zum Gebiet des chemischen Apparatebaus und kann insbesondere für die Durchführung der Synthese von Methylformiat durch Carbonylierung von Methanol angewandt werden.

Die Durchführung des industriellen Prozesses für die Erzeugung von Methylformiat durch Carbonylierung von Methanol mit Kohlenmonoxid ist dadurch kompliziert, daß eine große Kontaktoberfläche für das Gas (Kohlenmonoxid) mit der Flüssigkeit (Methanol) bei erhöhtem Druck von 2,0 bis 4,0 MPa für das Erreichen eines hohen Umsatzes (DE-OS 2710726, Kl. C07C 69/06) notwendig ist.

In chemischen und benachbarten Gebieten der Industrie gibt es viele Reaktortypen für die Realisierung des Stoffaustausches und chemische Umsetzung in Gas-Flüssigkeits-Systemen.

Vom Standpunkt des wichtigsten Parameters, der die Effektivität solcher Reaktoren charakterisisert - der Kontaktoberfläche der Phasen - wird ein Apparat mit Zirkulationskreis, genauer, ein Reaktor mit Spiralrührer und Strömungsrohr (Diffusor) mit Ablenkungszone im Gehäuse (V. N. Sokolov, N. V. Domanski, "Gas-Flüssig-Reaktoren", Quelle "Maschinostrojenije" 1976, S. 121), als geeignet angesehen.

In solchen Reaktoren entsteht ein geschlossener Zirkulationskreis und der Spiralrührer erfüllt die Rolle einer "Saug"-Pumpe und eines lokalen Dispergators.

Die Unzulänglichkeit solcher Apparaturen liegt in der Schwierigkeit der Konstruktion, insbesondere bei den rotierenden Teilen und den Abdichtungselementen des Rührers, insbesondere bei erhöhtem Druck, und der Gasgehalt des Systems wird in Grenzen gehalten.

Bekannt sind Ausrüstungen für die Dispersion und Mischung von Gas-Flüssig-Systemen, in denen die Verdichtung der Gasphase erzielt wird durch das turbulente Pulsieren im Strom bei gleichzeitigem Passieren der Komponenten durch Öffnungen in einer Trennwand mit Löchern und der anschließenden Vermischung im Gehäuse (Ippon, Ipone J, Size reduction of bubbles by orifice mixer, Chem. Engng., 1980, 35, Nr. 7, p. 1571 bis 1579).

Trotz der Einfachheit der Methode zur Dispersion und der unkomplizierten Konstruktion erfordert eine solche Ausrüstung dennoch einen erhöhten Energieverbrauch bei geringer Effektivität der Vermischung, da ein wesentlicher Teil der Energie des Gas-Flüssigkeits-Stromes nicht ausgenutzt wird.

Bekannt ist ein Reaktortyp für die Erzeugung von Methylformiat, in dem die Dispersion des Kohlenmonoxids durch die Energie des zirkulierenden Stromes des flüssigen Reaktionsgemisches erfolgt (DE-OS 2710726).

In der dort aufgezeigten technischen Lösung erfolgt die Dispersion der Gasphase durch die Saugstrahlwirkung des Gases mit der Flüssigkeit in dem Mischer vom Typ des Venturi-Rohrs und durch eine turbulente Pulsierung im Strom bei gleichzeitigem Durchgang der Bestandteile durch eine Trennwand mit Löchern.

Eine solche Methode der Dispersion des Gases ist energetisch nicht sinnvoll, und zwar aus den gleichen Gründen wie im vorherigen Beipiel. Bei dieser Methode wird der Reaktor nicht voll ausgenutzt (der Nutzungskoeffizient des Reaktors ist nicht größer als 0,91).

In der technischen Lösung kommt der vorliegenden Erfindung eine Mischvorrichtung für verschiedene Medien am nächsten, die ausgerüstet ist mit einem Diffusor mit Axial-Gehäuse und mit einer Düse, deren Achse längsverbunden ist mit der Trennnwand, die das Gehäuse in zwei Kammern für die vorbereitende und endgültige Mischung trennt (Erfinderschein UdSSR Nr. 816525, Kl. B01f 5/04).

Der aufgezeigte Apparat ist hauptsächlich bestimmt für die Erzeugung von Wasser-Brennstoff-Emulsionen und enthält eine Reihe anderer Konstruktionsdetails, die im vorliegenden Fall nicht geeignet sind und die Konstruktion unnötig kompliziert machen (richtunggebende perforierte Blätter in der Kammer der Vorvermischung, tangentiale Einführung der Komponenten in die Kammer, perforierte Aufsätze am Eingang in die Düse und in den Ableitungsstrom).

Aufgabe der vorliegenden Erfindung ist die Vereinfachung der Konstruktion des Reaktors und Erhöhung des Durchsatzes für die Erzeugung von Methylformiat.

Die Lösung der Aufgabe wird erreicht durch einen senkrecht stehenden Rohr-Reaktor mit einer inneren Zirkulationsvorrichtung, ausgeführt nach dem Prinzip einer Saugstrahlvorrichtung (Ejektor) ohne Diffusionswirkung sowie durch eine vorzugsweise horizontal angebrachte Trennwand mit Öffnungen und eine Mischkammer, die im Gehäuse über der Trennwand in Form eines Rohres koaxial angebracht ist, welches am oberen Ende stirnseitig verschlossen und im oberen Teil der Mantelfläche perforiert ist.

In dem vorgeschlagenen Reaktor bildet sich der Arbeitsstrom des Gas-Flüssigkeits-Gemisches durch die Öffnungen der horizontalen Trennwand sowie des im Gehäuse koaxial angebrachten Rohres, welches oben stirnseitig verschlossen und im oberen Teil längs des Umfangs perforiert ist, was zur Herausbildung eines inneren Zirkulationskreises, zur Anreicherung der Flüssigkeitsphase (Methanol) mit Gas (CO) und der Entwicklung der Kontaktoberfläche der Phasen führt.

Im Ergebnis des höheren Anteils des Kohlenmonoxids im Reaktionsgemisch wird eine höhere Effektivität im Prozeß für die Erzeugung von Methylformiat erzielt.

In Fig. 1 ist der Vertikalschnitt des Reaktors abgebildet, in Fig. 2, 3, 4 die Vertikalschnitte von weiteren Varianten.

In Fig. 5 sind die Untersuchungsergebnisse dargestellt, die die Besonderheiten der Reaktoren in der Gasphase bei der Erprobung mit Modellgemischen (Luft-Wasser) bestätigen. In Fig. 6 ist die Kinetik der Methylformiatkonzentration bei der Erprobung des vorgeschlagenen Reaktortyps dargestellt, die bei der Erzeugung von Methylformiat durch Carbonylierung von Methanol erreicht werden.

Der Reaktor besteht aus dem Gehäuse 1, aus der horizontal liegenden Trennwand 2 mit den Öffnungen 20 und der Mischkammer 3, die senkrecht über der Trennwand 2 in Form eines Rohres 10 angebracht ist, wobei das obere Ende verschlossen ist und im oberen Teil des Rohres 10 eine Perforation 21 vorhanden ist.

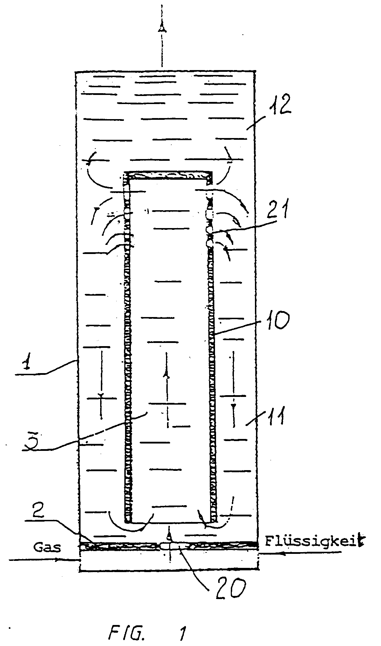

Der Reaktor (Fig. 1) arbeitet nach folgendem Prinzip:

Flüssigkeit und Gas treten unter der Trennwand 2 in den Reaktor ein. Beim gleichzeitigen Passieren der Öffnung 20 in der Trennwand 2 entsteht ein gebündelter Gas-Flüssigkeits-Strahl, dabei erfolgt die Zerteilung der Gasphase durch die turbulente Pulsierung, und der Strahl zieht das Gemisch aus dem ringförmigen Zwischenraum 11 zwischen dem Gehäuse 1 und dem Rohr 10 der Mischkammer 3. Der Mischstrom tritt in das Rohr 10 der Kammer 3 ein, dabei erfolgt durch den Ausgleich der Geschwindigkeiten ein Absinken des Druckes.

Nach Passieren der Perforation 21 in der Mischkammer 3 teilt sich der Strom. Der größere Teil des Stromes im ringförmigen Zwischenraum 11 zirkuliert zum unteren Teil des Rohrteiles 10 und der andere Teil des Stromes, entsprechend der mengenmäßigen Zuführung der Komponenten, tritt in den Raum 12 über dem oberen Ende des Rohres 10 der Mischkammer und tritt aus dem Reaktor aus.

Es bildet sich eine innere Zirkulation (Schlaufenströmung).

Bei der abrupten Umlenkung des Stromes beim Austritt aus der Mischkammer 3 in den ringförmigen Zwischenraum 11 erfolgt eine Trennung der leichten Phase zum Zentrum der Umlenkung, im Ergebnis derer in den zurückfließenden Strom ein sehr großer Teil der Gasphase, im Vergleich zum Hauptstrom, hineingerissen wird. Der zurückfließende Strom wird in Bezug auf die Gasphase bis zu dem Grenzwert des Gasinhaltes angereichert, der dem Phasengleichgewicht entspricht.

Die Gasphase in dem zurückfließenden Strom durchläuft mehrfach eine Zerteilung in der Mischkammer 3, im Ergebnis dessen der Dispersionsgrad aufrechterhalten wird.

Die Konstruktionselemente besitzen keine funktionellen Begrenzungen, die die Grenze des Gasinhaltes verringern.

Für die Organisation einer stabilen Dispersion von Gas in der Flüssigkeit bei sehr hohem Gasanteil im Strom ist von äußerster Wichtigkeit die Perforation 21 im oberen Teil des Rohres 10 der Mischkammer 3.

Bei der Vergrößerung der Durchmesser der Öffnungen über bestimmte Grenzwerte oder bei der Ausführung der Perforation 21 in Form von willkürlich angeordneten Öffnungen mit gleichem Durchmesser erzielt man nicht die erforderliche Dispersion der Gasphase bei hohem Gasanteil im Strom.

Aus diesem Grund wird die Perforation 21 oben am Rohr 10 in Form von strahlenförmig angeordneten Öffnungen mit Vergrößerungen des Durchmessers der Öffnungen von Reihe zu Reihe nach oben zunehmend ausgeführt. Am effektivsten ist die Perforation 21 des Rohres 10 beziehungsweise der Mischkammer 3 in Form von ringförmigen Schlitzen, wobei sich die Breite der Schlitze von Schlitz zu Schlitz nach oben zunehmend vergrößert.

Die Ausführung der Perforation 21 im oberen Teil des Rohres 10 in der Mischkammer in Form von Öffnungen, wo der Durchmesser von Reihe zu Reihe größer wird oder in Form von ringförmigen Schlitzen mit wachsender Breite, gewährleistet eine stabile Dispersion und Zirkulation des Gas-Flüssig-Gemisches bei Veränderungen des Durchsatzes von Gas und Flüssigkeit in sehr breitem Umfang.

In allen Fällen sind die Maße der Öffnungen und Schlitze eng mit anderen geometrischen Charakteristika des Reaktors und den Volumengeschwindigkeiten von Gas und Flüssigkeit verbunden. Diese Maße werden experimentell ermittelt.

Bei großem Durchmesser des Reaktorgehäuses kann man andere Varianten für die Zirkulationsvorrichtungen, basierend auf dem gleichen Prinzip der Dispersion von Gas in Flüssigkeit, wie in der Grundvariante (Fig. 1) anwenden.

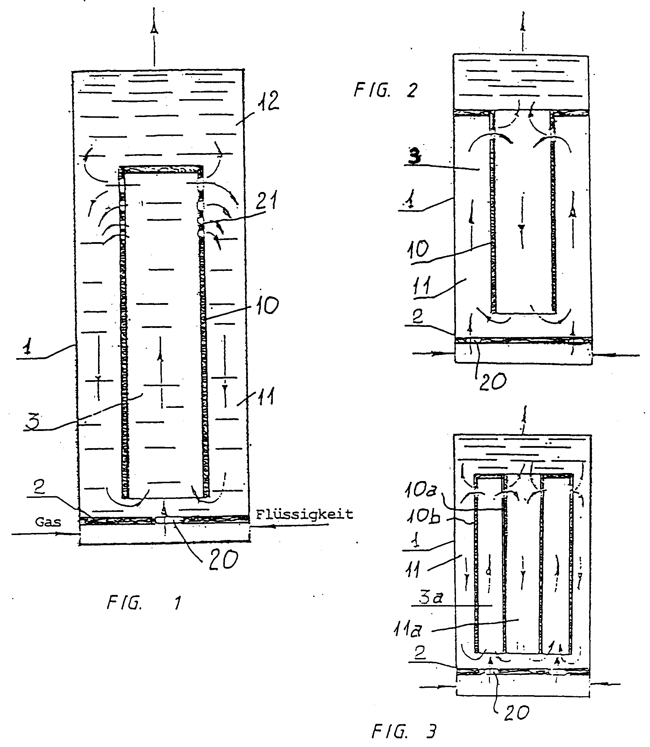

In der Fig. 2 ist eine Variante aufgezeigt, in der die Trennwand 2 eine Reihe von Öffnungen 20 besitzt, die unter dem ringförmigen Zwischenraum 11 zwischen Gehäuse 1 und der rohrförmigen Mischkammer 3 liegen.

Hier erfüllt der ringförmige Raum zwischen Gehäuse 1 und Rohr 10 die Funktion der Mischkammer 3. Aus diesem Grunde wird nicht der obere Teil des Rohres 10 verschlossen, sondern der ringförmige Zwischenraum 11 zwischen dem oberen Teil des Rohres 10 und dem Gehäuse 1.

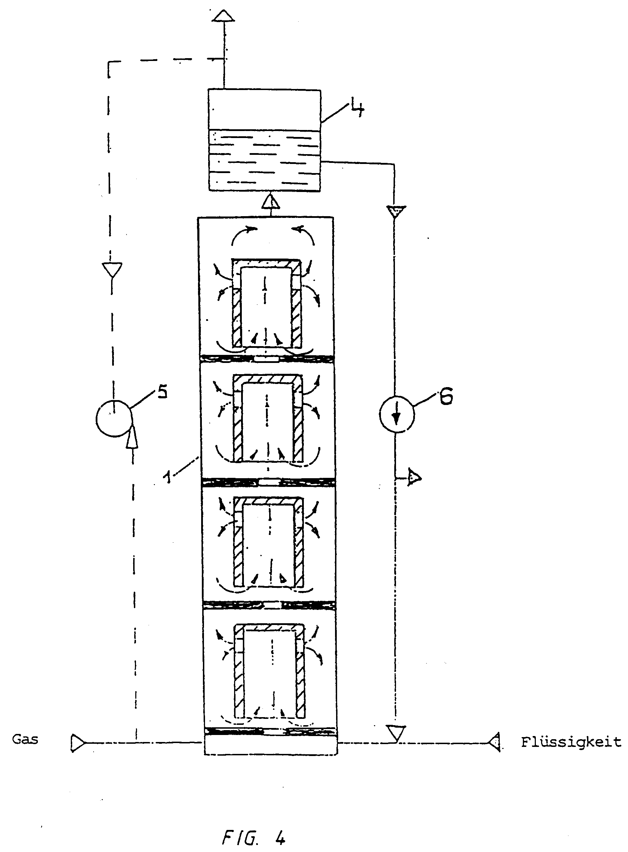

In Fig. 3 ist die Mischkammer 3 in Form von zwei konzentrisch angeordneten Rohren unterschiedlichen Durchmessers ausgeführt, wobei der obere ringförmige Zwischenraum zwischen dem äußeren und inneren Rohr 10b, 10a geschlossen ist, und die oberen Teile des äußeren und des inneren Rohres sind perforiert.

Die Öffnungen 20 in der Trennwand 2 sind ausgeführt in Form einer Reihe von Durchlaßkanälen, die unter dem ringförmigen Zwischenraum des Doppelrohres (10a, 10b) liegen. Hierdurch zirkuliert das Fluid in einer Art doppelter Schlaufenströmung.

In der dargestellten Variante (Fig. 3) erfüllt der ringförmige Zwischenraum 3a zwischen den Koaxial-Rohren 10a, 10b die Funktion der Mischkammer.

Die dargestellte Variante des Einkammer-Reaktors (Fig. 1 bis 3) gestattet einen hohen Gasanteil im Reaktionsgemisch und die Entwicklung einer Kontaktoberfläche für Gas und Flüssigkeit, jedoch keine ausreichend lange Kontaktzeit. Zur Verlängerung der Kontaktzeit kann man einen Reaktor mit aufeinanderfolgender Anordnung der Zirkulationsbereiche anwenden (Fig. 4). In jedem Bereich erfolgt der Kontakt des Gases mit der Flüssigkeit analog dem oben beschriebenen Vorgang.

Der Gas-Flüssigkeits-Strom durchläuft alle Bereiche und tritt dann in den Abscheidebehälter 4 ein, wo die Phasentrennung erfolgt. Bei solcher Ausführung stellt der Reaktor eine Kaskade von Reaktoren mit idealer Vermischung dar, zumal die Verweilzeit mit der Anzahl der Zirkulationsbereiche wächst.

Für die Herstellung einer stabilen Dispersion und Zirkulation des Stromes ist eine ziemlich hohe Volumengeschwindigkeit von Gas und Flüssigkeit erforderlich, was einer Erhöhung der Verweilzeit entgegenwirkt, wenn man diese durch Erhöhung der Anzahl der Zirkulationsvorrichtungen bei einfachem Durchgang des Stromes (ohne Rückführung) erzielen will.

Eine weitere Erhöhung der Verweilzeit erzielt man durch die Anwendung einer äußeren Zirkulation der Flüssigkeits- und Gasphase (bei Erfordernis). Damit erreicht man aufgrund der äußeren Zirkulation ein stabiles hydrodynamisches Arbeitsregime des Reaktors, und die Verweilzeit hängt hier nur von den Volumengeschwindigkeiten der in das Reaktorsystem eintretenden Gase und Flüssigkeiten ab, die in einem breiten Umfang variiert werden können.

Die Kaskadenvariante des Reaktors (Fig. 4) sieht die Möglichkeit einer äußeren Zirkulation des Gases aus dem Abscheidebehälter 4 in den Reaktor 1 mit Hilfe des Kompressors 5 und der Flüssigkeit mit Pumpe 6 vor.

Bei allen oben beschriebenen Ausführungsbeispielen der Reaktoren (Fig. 1 bis 4) gibt es für die Gewährleistung der inneren Zirkulation ein und dieselbe Lösung: das Verschließen des oberen Endes der Mischkammer, die Perforierung im oberen Teil der Mischkammer und die Trennwand mit Öffnungen direkt unter der Mischkammer, die dafür sorgen, daß sich das Gas-Flüssigkeits-Gemisch ausbildet. Die Richtung des Stoffstromes in den verschiedenen Varianten ist in Fig. 1, 2, 3 und 4 durch Pfeile angedeutet.

Die Konstruktion des vorgeschlagenen Reaktors sowie der Varianten ist wesentlich einfacher als die Konstruktion des "Prototyp-Reaktors". Im Zusammenhang damit gestattet der Reaktor in Übereinstimmung mit der Erfindung einen hohen Gasanteil im Gas-Flüssigkeits-Gemisch und die Entwicklung der Kontaktoberfläche der Phasen. Das wurde bestätigt durch die Resultate der experimentellen Untersuchungen an einem Reaktor mit einer Zirkulationsvorrichtung.

Als Modellsubstanzen wurden Wasser und Luft gewählt.

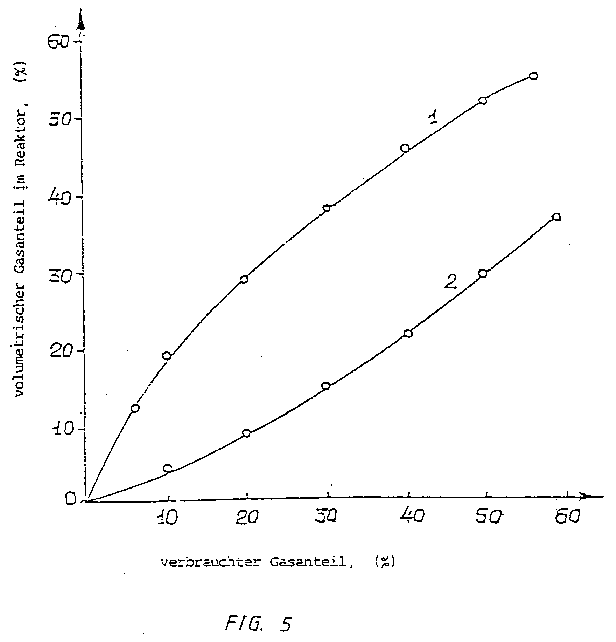

Aus der graphischen Darstellung (Fig. 5) ist eine wesentliche Erhöhung des Gasinhaltes im erfindungsgemäßen Reaktor mit Zirkulationsvorrichtung (Kurve 1), im Vergleich zu einem Reaktor gemäß Prototyp ohne Mischkammer, mit perforierter Trennwand und einer Phasenvermischung (Kurve 2) ersichtlich. Bei gleichem Durchsatz des Gasinhaltes (zum Beispiel 20 Prozent) ist der volumenmäßige Gasinhalt im Fall des vorgeschlagenen Reaktors um 1,5 bis 5 mal höher (um 3,5 mal in dem dargestellten Beispiel) als mit dem Reaktor ohne Zirkulationsvorrichtung.

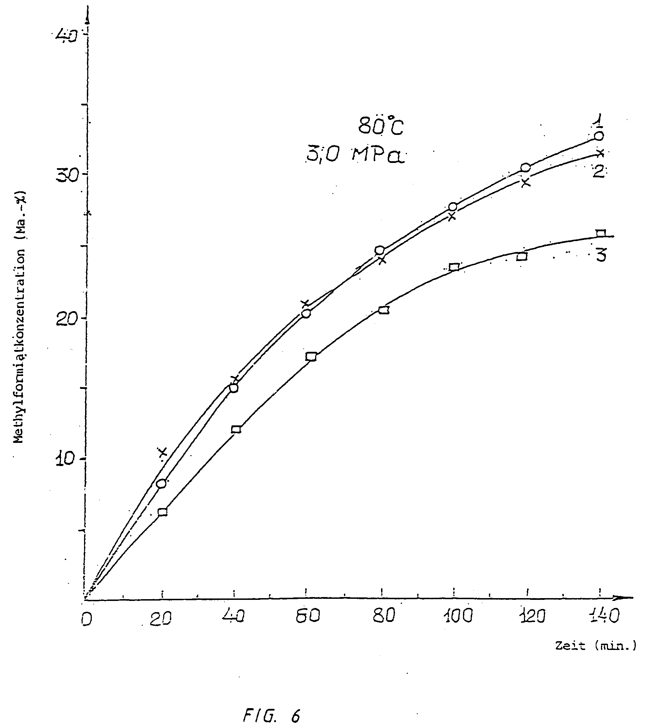

Bei der Nutzung des erfindungsgemäßen Reaktors nach Fig. 4, der einige (3 bis 12) aufeinanderfolgende Zirkulationsvorrichtungen enthält, im Prozeß der Methylformiatsynthese erreicht man einen hohen Anteil von CO in der Methanollösung und die Ausbildung einer Kontaktoberfläche der Phasen, ausreichend für den Verlauf des Prozesses im kinetischen oder nahezu kinetischen Bereich. Diese Aussage wird gestützt durch den Vergleich der Konzentrations-Kurven von Methylformiat (Fig. 6) bei der Durchführung des Prozesses in dem erfindungsgemäßen Reaktor (Kurve 1) und im Reaktor mit dem Spiralrührer, der die Diffusion ausrichtet und der Ablenkungszone im Gehäuse (Kurve 2), der im kinetischen Bereich arbeitet. Aus den dargestellten Kurven wird ersichtlich, daß die Geschwindigkeiten zur Anreicherung des Methylformiats bei der Anwendung des erfindungsgemäßen Reaktors und des Reaktors mit Spiralrührer, der im kinetischen Bereich arbeitet, ähnlich sind.

Die Kurve 3 (Fig. 6) stellt die Experimentaldaten für die Anreicherung des Methylformiates durch Carbonylierung von Methanol mit einem Gasgemisch, das nur zu 75 Prozent aus CO besteht, in dem vorgeschlagenen Reaktor dar. Auch in diesem Fall beobachet man eine hohe Geschwindigkeit bei der Anreicherung des Produktes.

Ein wichtiger und wesentlicher Vorteil des vorgeschlagenen Reaktors besteht daher in der Möglichkeit der Anwendung einer niedrigen Konzentration des CO für die Carbonylierung des Methanols.

Ein Beispiel für die Durchführung des Prozesses der Synthese von Methylformiat wird nachfolgend aufgeführt.

### Beispiel 1

Die Synthese von Methylformiat durch Carbonylierung von Methanol erfolgt im Reaktor (Fig. 4) mit vier aufeinanderfolgenden Zirkulationsvorrichtungen nach Fig. 1, Abscheidebehälter 7 und Zirkulationspumpe 6. Für die Abführung der Reaktionswärme ist der Reaktor mit einem thermostatischen Mantel ausgerüstet. Die Länge der Zirkulationsvorrichtung (von Trennwand zu Trennwand) beträgt 400 mm, die inneren Durchmesser für das röhrenförmige Gehäuse 50 mm, für die Mischkammer 34 mm.

Das Volumen des Reaktors ist 4,14 dm³, der Koeffizient der Füllung beträgt 0,96. Zur Gewährleistung eines stabilen Arbeitsregimes werden die flüssigen Produkte aus dem Abscheidebehälter 4 mit der Pumpe 6 zum Reaktoreingang zurückgeführt.

Der Prozeß wird bei einem Druck von 4 MPa und einer Temperatur von 80 +/- 5 Grad Celsius durchgeführt.

Am Reaktoreingang werden kontinuierlich 1,885 Kilogramm/Stunde (1,508 Nm³/Stunde) 99,2 prozentiges Kohlenmonoxid (Rest sind inerte Gase) und 8,232 Kilogramm/Stunde 2,5 Massen-Prozent Natriummethylat in einer Methanollösung aufgegeben.

Gleichzeitig fließen 510 Kilogramm/Stunde Reaktionsflüssigkeit aus dem Abscheidebehälter 4 zum Eingang des Reaktors zurück.

Aus dem oberen Teil des Abscheidebehälters 4 werden ständig 0,080 Nm³/Stunde (0,100 Kilogramm/Stunde) des nachfolgenden Gasgemisches in das Abgassystem abgeführt:

85,0 Masse-% (0,085 Kilogramm/Stunde) Kohlenmonoxid
15,0 Masse-% (0,015 Kilogramm/Stunde) Stickstoff und andere inerte Gase

Aus dem Abscheidebehälter werden 10,017 Kilogramm/Stunde Produkt mit folgender Zusammensetzung (chromatographische und chemische Analyse) abgeführt:
60,59 Masse-% (6,070 Kilogramm/Stunde) Methanol
36,74 Masse-% (3,680 Kilogramm/Stunde) Methylformiat
0,49 Masse-% (0,049 Kilogramm/Stunde) Kohlenmonoxid
1,72 Masse-% (0,172 Kilogramm/Stunde) Natriummethylat
0,46 Masse-% (0,046 Kilogramm/Stunde) Zerfallsprodukte von Natriummethylat

Nach der Rektifikation der Produkte erhält man 3,675 Kilogramm/Stunde Methylformiat.

Die Ergiebigkeit von 1 Liter Reaktionsgemisch beträgt 1,170 Kilogramm/Stunde Methylformiat, der Umsatz (beim Durchgang) von Kohlenmonoxid 94,7 Prozent, die Umwandlung von Methanol 24,48 Prozent.

Das Beispiel zur Nutzung des Reaktors für die Erzeugung von Methylformiat zeigt, daß man durch die Anwendung der vorgeschlagenen technischen Lösungen eine hohe Ergiebigkeit im Prozeß für die Erzeugung von Methylformiat erzielt. Die erzielte Ergiebigkeit (1,170 Kilogramm/Stunde Methylformiat) ist um 44,4 Prozent höher als bei analogen Erfindungen, trotz gleicher Bedingungen (Beispiel in DE-OS 2710726).

Beipiele für die Anwendung komplizierterer Reaktortypen als den Reaktor-Prototyp im Prozeß der Methylformiatsynthese sind nicht bekannt.

Somit kann festgestellt werden, daß der vorgeschlagene Reaktor eine einfache Konstruktion hat, keine rotierenden Teile enthält, die Entstehung einer Kontaktoberfläche für Kohlenmonoxid und Methanol und einen höheren Gasanteil (bis 50 Prozent) im Gas-Flüssig-Strom gewährleistet, die Durchführung eines sehr ergiebigen Prozesses für die Methylformiatsynthese durch Carbonylierung von Methanol (Erzeugung von 1,17 Kilogramm/Stunde Methylformiat je 1 Liter) ermöglicht und die Verwendung von Kohlenmonoxid mit geringerer Konzentration (sogar 75 Masse-Prozent) gestattet.

Die Konstruktion der Zirkulationsvorrichtungen bietet die Möglichkeit, durch die Verbindung aufeinanderfolgender einheitlicher Elemente einen Reaktor mit dem erforderlichen Fassungsvermögen beziehungsweise die erforderliche Anzahl von Kaskaden (Stufen für die Veränderung der Konzentration) zu erhalten.

Besonders geeignet ist der vorgeschlagene Reaktortyp für die Realisierung von Reaktionen in Gas-Flüssigkeits-Systemen bei hohen Drücken.

## Patentansprüche

1. Reaktorkonstruktionen insbesondere für die Methylformiatsynthese, bestehend aus einem rohrförmigen Gehäuse, in dem Zirkulationseinrichtungen enthalten sind, dadurch gekennzeichnet, daß zur Vereinfachung der Konstruktion und zur Erhöhung der Ergiebigkeit bei der Erzeugung von Methylformiat
- die Zirkulationseinrichtung nach dem Prinzip einer Saugstrahlvorrichtung (Ejektor) ausgebildet ist,
- das Gehäuse (1) unten eine Trennwand (2) enthält, unter der Gas und Flüssigkeit zugeleitet werden,
- die Trennwand (2) eine oder mehrere Öffnungen (20) enthält,
- oberhalb der Trennwand (2) und der Öffnungen (20) eine Mischkammer (3, 3a) innerhalb eines Rohres (10) oder mehrerer koaxialer Rohre (10a, 10b) angeordnet ist,
- die Mischkammer (3, 3a) oben stirnseitig verschlossen ist und
- die die Mischkammer (3, 3a) bildenden Rohre (10, 10a, 10b) im oberen Bereich am Umfang perforiert sind.

2. Reaktor nach Anspruch 1, dadurch gekennzeichnet, daß die Perforationen (21) in der Mischkammer (3, 3a) in Form von Reihen radialer Öffnungen ausgestaltet sind, wobei sich der Durchmesser der Öffnungen von Reihe zu Reihe nach oben hin vergrößert.

3. Reaktor nach Anspruch 1, dadurch gekennzeichnet, daß die Perforationen (21) in der Mischkammer (3, 3a) als ringförmige Schlitze mit nach oben hin zunehmender Breite ausgeführt sind.

4. Reaktor nach Anspruch 1, dadurch gekennzeichnet, daß die Mischkammer (3) innerhalb des Rohres (10) ersetzt ist durch eine ringförmige Mischkammer (3), die zwischen dem rohrförmigen Gehäuse (1) und dem Innenrohr (10) angeordnet ist und die Öffnungen (20) in der horizontalen Trennwand (2) in Form von Durchgangskanälen ausgeführt sind, die sich unterhalb des ringförmigen Zwischenraums (11) zwischen dem äußeren Gehäuse (1) und dem Innenrohr (10) befinden, wobei das obere Ende des Rohres (10) offen und der Zwischenraum (11) zwischen oberem Ende des Rohres (10) und dem Gehäuse (1) verschlossen ist.

5. Reaktor nach Anspruch 1, dadurch gekennzeichnet, daß eine ringförmige Mischkammer (3a) zwischen koaxialen Rohren (10a, 10b) gebildet ist, der Zwischenraum zwischen den oberen Enden der Rohre (10a, 10b) verschlossen ist, die Öffnungen (20) in Form von Durchgangskanälen in der Platte (2) unter dem Zwischenraum zwischen den Rohren (10a, 10b) angeordnet sind, so daß eine aufwärts gerichtete Strömung in der ringförmigen Mischkammer (3a) erzeugt werden kann und abwärts gerichtete Ströme in dem inneren Rohr (10a) und außerhalb des äußeren Rohres (10b) bewirkt werden können.

6. Reaktor nach einem oder mehreren der Ansprüche 1 bis 5, dadurch gekennzeichnet, daß in einem rohrförmigen Gehäuse (1) eine Reihe von Zirkulationsvorrichtungen, bestehend aus Trennwand (2) und Mischkammern (3, 3a) enthaltenden Rohren (10, 10a, 10b), nacheinander in Form einer Kaskade angeordnet sind.

7. Reaktor nach einem oder mehreren der Ansprüche 1 bis 6, dadurch gekennzeichnet, daß zur Erhöhung der Verweil- und Kontaktzeit eine äußere Zirkulation aus einem Abscheidebehälter (4) in den Raum unter einer horizontalen Trennwand (2) für die Flüssigkeit über eine Pumpe (6) und/oder für das Gas über einen Kompressor (5) vorgesehen ist.

## Claims

1. Reactor constructions, more especially for methyl formate synthesis, comprising a tubular housing which contains circulation arrangements, characterised in that, to simplify the construction and to increase the yield when producing methyl formate,
- the circulation arrangement is configured according to the principle of an ejector,
- the housing (1) is provided at its lower end with a dividing wall (2), beneath which gas and liquid are supplied,
- the dividing wall (2) has one or more apertures (20),
- a mixing chamber (3, 3a) is disposed above the dividing wall (2) and the apertures (20) internally of a tube (10) or a plurality of coaxial tubes (10a, 10b),
- the mixing chamber (3, 3a) is sealed at its upper end face, and
- the tubes (10, 10a, 10b), forming the mixing chamber (3, 3a), are perforated in the upper region on the periphery.

2. Reactor according to claim 1, characterised in that the perforations (21) in the mixing chamber (3, 3a) are configured in the form of rows of radial apertures, the diameter of the apertures increasing upwardly from row to row.

3. Reactor according to claim 1, characterised in that the perforations (21) in the mixing chamber (3, 3a) are configured as annular slots having a width which increases upwardly.

4. Reactor according to claim 1, characterised in that the mixing chamber (3) internally of the tube (10) is replaced by an annular mixing chamber (3), which is disposed between the tubular housing (1) and the inner tube (10), and the apertures (20) in the horizontal dividing wall (2) are configured in the form of through-ducts which are situated beneath the annular space (11) between the outer housing (1) and the inner tube (10), the upper end of the tube (10) being open, and the space (11) between the upper end of the tube (10) and the housing (1) being sealed.

5. Reactor according to claim 1, characterised in that an annular mixing chamber (3a) is formed between coaxial tubes (10a, 10b), the space between the upper ends of the tubes (10a, 10b) is sealed, and the apertures (20), in the form of through-ducts in the wall (2), are disposed beneath the space between the tubes (10a, 10b), so that an upwardly directed flow can be produced in the annular mixing chamber (3a), and downwardly directed streams can be produced in the inner tube (10a) and externally of the outer tube (10b).

6. Reactor according to one or more of claims 1 to 5, characterised in that a number of circulation arrangements, comprising tubes (10, 10a, 10b) which contain dividing wall (2) and mixing chambers (3, 3a), are disposed successively in the form of a cascade in a tubular housing (1).

7. Reactor according to one or more of claims 1 to 6, characterised in that, in order to increase the dwell time and contact time, an external circulation from a separator (4) into the space beneath a horizontal dividing wall (2) is provided for the liquid via a pump (6) and/or for the gas via a compressor (5).

## Revendications

1. Structures de réacteur, en particulier pour la synthèse du formiate de méthyle, constituées d'un boîtier tubulaire contenant des dispositifs de recirculation, caractérisées en ce que, pour simplifier la construction et augmenter le rendement lors de la production de formiate de méthyle:
- le dispositif de recirculation est configuré suivant le principe d'un dispositif d'aspiration et de projection (éjecteur),
- le boîtier (1) contient dans le bas une paroi de séparation (2) en dessous de laquelle le gaz et le liquide sont amenés,
- la paroi de séparation (2) contient une ou plusieurs ouvertures (20),
- au-dessus de la paroi de séparation (2) et des ouvertures (20), une chambre de mélange (3, 3a) est disposée à l'intérieur d'un tube (10) ou de plusieurs tubes coaxiaux (10a, 10b),
- la chambre de mélange (3, 3a) est fermée frontalement dans le haut, et
- dans la partie supérieure, les tubes (10, 10a, 10b) formant la chambre de mélange (3, 3a) sont perforés à leur périphérie.

2. Réacteur selon la revendication 1, caractérisé en ce que les perforations (21) dans la chambre de mélange (3, 3a) sont réalisées sous la forme de séries d'ouvertures radiales, le diamètre des ouvertures augmentant vers le haut d'une série à l'autre.

3. Réacteur selon la revendication 1, caractérisé en ce que les perforations (21) dans la chambre de mélange (3, 3a) sont réalisées sous la forme de fentes annulaires dont la largeur augmente vers le haut.

4. Réacteur selon la revendication 1, caractérisé en ce que la chambre de mélange (3) à l'intérieur du tube (10) est remplacée par une chambre de mélange (3) de forme annulaire qui est disposée entre le boîtier (1) de forme tubulaire et le tube intérieur (10), et les ouvertures (20) dans la paroi horizontale de séparation (2) sont réalisées sous la forme de canaux de traversée qui sont situés en dessous de l'espace intermédiaire (11) de forme annulaire situé entre le boîtier extérieur (1) et le tube intérieur (10), l'extrémité supérieure du tube (10) étant ouverte et l'espace intermédiaire (11) entre l'extrémité supérieure du tube (10) et le boîtier (1) étant fermé.

5. Réacteur selon la revendication 1, caractérisé en ce qu'une chambre de mélange (3a) annulaire est formée entre des tubes coaxiaux (10a, 10b), l'espace intermédiaire entre les extrémités supérieures des tubes (10a, 10b) est fermé et les ouvertures (20) présentant la forme de canaux de traversée sont disposées dans la plaque (2) en dessous de l'espace intermédiaire situé entre les tubes (10a, 10b), de sorte que l'on peut créer dans la chambre de mélange (3a) de forme annulaire un écoulement dirigé vers le haut, et que l'on peut réaliser dans le tube intérieur (10a) et à l'extérieur du tube extérieur (10b) des écoulements dirigés vers le bas.

6. Réacteur selon l'une ou plusieurs des revendications 1 à 5, caractérisé en ce que dans un boîtier (1) de forme tubulaire, une série de dispositifs de recirculation, constitués de la paroi de séparation (2) et de tubes (10, 10a, 10b) contenant des chambres de mélange (3, 3a), sont disposés l'un derrière l'autre en forme de cascade.

7. Réacteur selon l'une ou plusieurs des revendications 1 à 6, caractérisé en ce qu'en vue d'augmenter le temps de séjour et de contact, il est prévu une circulation extérieure depuis un réservoir de séparation (4) jusque dans l'espace situé en dessous d'une paroi horizontale de séparation (2), par l'intermédiaire d'une pompe (6) pour le liquide, et/ou par l'intermédiaire d'un compresseur (5) pour le gaz.
